# EUROPEAN PATENT APPLICATION

(11) **EP 3 922 315 A1**
(43) Date of publication of application: **15.12.2021**
(21) Application number: 20752678.1
(22) Date of filing: 10.01.2020
(51) Int. Cl.: A61Q 19/00, A61K 8/02, A61K 8/04, A61K 8/31, A61K 8/34, A61K 8/55, A61K 8/64, B01J 13/00

(54) **METHOD FOR PRODUCING GELATINOUS COMPOSITION**

(30) Priority: 05.02.2019 JP 2019018630
(71) Applicant: KANEKA CORPORATION, Osaka 530-8288 (JP)
(72) Inventor: TSUJI, Tadao, Settsu-shi, Osaka 566-0072 (JP); YANAGISAWA, Satohiro, Tokyo 107-6028 (JP)
(74) Representative: Hoffmann Eitle
(86) International application number: PCT/JP2020/000659
(87) International publication number: WO 2020/162103

(57) **Abstract**

The objective of the present invention is to provide a method for easily producing a gelatinous composition that contains a phospholipid and is excellent in an emulsion stability, a method for easily producing an emulsified composition excellent in a use feeling and an emulsion stability, a gelatinous composition excellent in an emulsion stability, and an emulsified composition excellent in a use feeling and an emulsion stability. The method for producing a gelatinous composition according to the present invention is characterized in comprising the steps of dissolving a phospholipid and a cyclic lipopeptide biosurfactant in a polyol or a mixed solvent of water and a polyol to obtain a surfactant solution, and mixing an oily ingredient with the surfactant solution to obtain the gelatinous composition, wherein a mass ratio of the cyclic lipopeptide biosurfactant to the phospholipid in the gelatinous composition is 0.25 or more.

## Description

### TECHNICAL FIELD

The present invention relates to a method for easily producing a gelatinous composition excellent in an emulsion stability, a method for easily producing an emulsified composition excellent in a use feeling and an emulsion stability, a gelatinous composition excellent in an emulsion stability, and an emulsified composition excellent in a use feeling and an emulsion stability.

### BACKGROUND ART

An aqueous ingredient and an oily ingredient cannot be inherently mixed each other. An emulsified composition prepared by stably mixing an aqueous ingredient and an oily ingredient with a surfactant is utilized in various fields. Various mixing methods therefor have been developed, and a mechanical stirring method is generally used.

An example of such a mechanical stirring method includes a method using a mixing device such as a homo-mixer and a disperser mixer, and a method using a filter such as a filter mixer. An emulsified composition can be obtained by mainly giving a strong stirring force and strong shear to a particle to be pulverized by a mechanical stirring method. A mechanical stirring method, however, has the disadvantages that the method requires a process such as increasing temperature, evacuation and cooling, consumes a large amount of energy such as electric power, and cannot be interrupted.

An example of a method for producing an emulsified composition other than a mechanical stirring method includes a D phase emulsification method and a liquid crystal emulsification method. A D phase emulsification method is a method for obtaining an O/W emulsion by dissolving a surfactant in water and a polyol to obtain a solution, dispersing an oily ingredient in the solution to obtain an O/D gel emulsion, and adding water to the O/D gel emulsion (Patent document 1 and Non-patent document 1). A liquid crystal emulsification method is a method for obtaining an O/W emulsion by, for example, dispersing an oil phase to be retained in a lamellar liquid crystal of a surfactant to form a gelatinous O/LC emulsion and adding water to the O/LC emulsion (Patent document 2).

When an emulsion is produced by the above methods, a compounding ratio and a compounding condition of each ingredient are extremely limited. For example, a use feeling of the emulsion is bad, since a compounding amount of an oily ingredient must be relatively small and a compounding amount of a surfactant must be relatively large.

A phospholipid is exemplified as an amphipathic ingredient that has a surface activity and additionally gives an excellent use feeling. A phospholipid is an important constituent of a cell membrane and forms a lipid bilayer together with a glycolipid and cholesterol. An emulsified composition containing a phospholipid is, therefore, excellent in conformability to the skin and used as an ingredient for a skin external agent, cosmetics or the like.

### PRIOR ART DOCUMENT

### PATENT DOCUMENT

Patent document 1: JP S63-072335 A
Patent document 2: JP H9-124432 A

### NON-PATENT DOCUMENT

Non-patent document 1: SAGITANI Hiromichi et al., Journal of Japan Oil Chemist's Society, Vol. 40, No. 11, pp. 988-994 (1991)

### DISCLOSURE OF THE INVENTION

### PROBLEMS TO BE SOLVED BY THE INVENTION

A phospholipid is a very important ingredient for an emulsified composition, since a phospholipid has a surface activity and additionally gives an excellent use feeling as described above. On the one hand, a phospholipid has the disadvantages that a surface activity thereof is not sufficient and an emulsion stability of an emulsified composition containing a phospholipid is not sufficient.

The objective of the present invention is to provide a method for easily producing a gelatinous composition that contains a phospholipid and is excellent in an emulsion stability, a method for easily producing an emulsified composition excellent in a use feeling and an emulsion stability, a gelatinous composition excellent in an emulsion stability, and an emulsified composition excellent in a use feeling and an emulsion stability.

### MEANS FOR SOLVING THE PROBLEMS

The inventors of the present invention repeated intensive studies in order to solve the above-described problems. As a result, the inventors completed the present invention by finding that a gelatinous composition excellent in an emulsion stability and an emulsified composition excellent in a use feeling and an emulsion stability can be easily produced by using the specific biosurfactant in addition to a phospholipid and adjusting the ratios thereof.

The present invention is hereinafter described.

[1] A method for producing a gelatinous composition, the method comprising the steps of:
   dissolving a phospholipid and a cyclic lipopeptide biosurfactant in a polyol or a mixed solvent of water and a polyol to obtain a surfactant solution, and
   mixing an oily ingredient with the surfactant solution to obtain the gelatinous composition,
   wherein a mass ratio of the cyclic lipopeptide biosurfactant to the phospholipid in the gelatinous composition is 0.25 or more.

The method according to the above [1], wherein the phospholipid is one or more phospholipids selected from phosphatidylcholine, hydrogenated lecithin, phosphatidic acid, bisphosphatidic acid, phosphatidylethanolamine, phosphatidylmethylethanolamine, phosphatidylserine, phosphatidylinositol, phosphatidylglycerol, diphosphatidylglycerol and sphingomyelin.

The method according to the above [1] or [2], wherein the cyclic lipopeptide biosurfactant is one or more cyclic lipopeptide biosurfactants selected from surfactin, arthrofactin, iturin and salts thereof.

The method according to any one of the above [1] to [3], wherein a total ratio of the phospholipid and the cyclic lipopeptide biosurfactant in the gelatinous composition is 0.01 mass% or more and 10 mass% or less.

The method according to any one of the above [1] to [4], wherein a ratio of the oily ingredient in the gelatinous composition is 1 mass% or more and 90 mass% or less.

The method according to any one of the above [1] to [4], wherein a ratio of the oily ingredient in the gelatinous composition is 1 mass% or more and less than 50 mass%.

A method for producing an emulsified composition, the method comprising the steps of:
producing the gelatinous composition by the method according to any one of the above [1] to [6], and
dispersing the gelatinous composition in an aqueous solvent.

A gelatinous composition,
comprising a phospholipid and a cyclic lipopeptide biosurfactant,
wherein a mass ratio of the cyclic lipopeptide biosurfactant to the phospholipid is 0.25 or more.

An emulsified composition,
comprising a phospholipid, a cyclic lipopeptide biosurfactant and an aqueous solvent,
wherein a mass ratio of the cyclic lipopeptide biosurfactant to the phospholipid is 0.25 or more.

### EFFECT OF THE INVENTION

A gelatinous composition that contains a phospholipid but is excellent in an emulsion stability can be easily produced by the present invention method. In addition, an emulsified composition excellent in a use feeling and an emulsion stability can be easily produced by dispersing the gelatinous composition in an aqueous solvent. The present invention is, therefore, industrially very excellent as a method for producing an emulsified composition useful as a skin external agent, cosmetics or the like.

### MODE FOR CARRYING OUT THE INVENTION

Hereinafter, the method for producing a gelatinous composition according to the present invention is first described with the specific example. The present invention is not restricted to the following embodiment.

### 1. Step for preparing surfactant solution

A phospholipid and a cyclic lipopeptide biosurfactant are dissolved in a polyol or a mixed solvent of water and a polyol to prepare a surfactant solution in this step.

A phospholipid is a general term for a lipid that has a phosphate ester part in the structure, and is classified into a glycerophospholipid containing glycerin in the skeleton and a sphingophospholipid containing sphingosine in the skeleton. Sphingosine has a structure corresponding glycerin of which hydroxy group at C2 position is replaced with an amino group and to which a long chain alkyl group is bound at C1 position. The phosphate group forms a phosphate ester with a hydroxy group of glycerin or sphingosine. A gelatinous composition and an emulsified composition containing a phospholipid has an excellent use feeling, since a phospholipid is one of structural components of a cell membrane.

An example of a phospholipid is represented by the following formula (I): 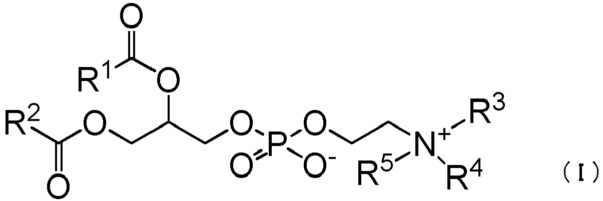 wherein R¹ and R² are independently a C₈₋₂₄ alkyl group, a C₈₋₂₄ alkenyl group or a C₈₋₂₄ alkynyl group, and R³ to R⁵ are independently a C₁₋₆ alkyl group.

A C₈₋₂₄ alkyl group means a linear or branched monovalent saturated aliphatic hydrocarbon group having a carbon number of 8 or more and 24 or less. An example of the C₈₋₂₄ alkyl group includes octyl, nonyl, decyl, dodecyl, tetradecyl, hexadecyl, octadecyl, eicosyl, docosyl, isotetradecyl, isohexadecyl, isooctadecyl, isoeicosyl, isodocosyl, 2-butyldecyl, 2-hexyldecyl, 2-octyldecyl, 2-decanyldecyl, 2-dodecanyldecyl and tetracosyl.

A C₈₋₂₄ alkenyl group means a linear or branched monovalent unsaturated aliphatic hydrocarbon group that has a carbon number of 8 or more and 24 or less and that has at least one carbon-carbon double bond.

A C₈₋₂₄ alkynyl group means a linear or branched monovalent unsaturated aliphatic hydrocarbon group that has a carbon number of 8 or more and 24 or less and that has at least one carbon-carbon triple bond.

A C₁₋₆ alkyl group means a linear or branched monovalent saturated aliphatic hydrocarbon group having a carbon number of 1 or more and 6 or less. An example of the C₁₋₆ alkyl group includes methyl, ethyl, n-propyl, isopropyl, n-butyl, isobutyl, s-butyl, t-butyl, n-pentyl and n-hexyl. The C₁₋₆ alkyl group is preferably a C₁₋₄ alkyl group, more preferably a C₁₋₂ alkyl group, and even more preferably methyl.

A phospholipid is specifically exemplified by lecithin. Lecithin is another name for phosphatidylcholine having the following structure.

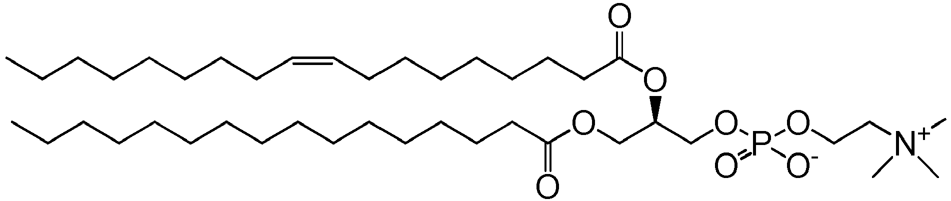

A naturally occurring lipid product containing a phospholipid is called as natural lecithin in some cases. For example, a phospholipid product derived from egg yolk is called as egg yolk lecithin and a phospholipid product derived from soybean is called as soybean lecithin in some cases.

In addition, an example of a phospholipid includes a glycerophospholipid such as phosphatidic acid, bisphosphatidic acid, phosphatidylserine, phosphatidylethanolamine, phosphatidylmethylethanolamine, phosphatidylinositol, phosphatidylglycerol and diphosphatidylglycerol; a sphingophospholipid such as sphingosine, ceramide, sphingomyelin and cerebroside; a hydrogenated lecithin such as hydrogenated soybean phospholipid and hydrogenated egg yolk lecithin.

A ratio of a phospholipid in the gelatinous composition may be appropriately adjusted and may be adjusted to, for example, 0.02 mass% or more and 10 mass% or less to the gelatinous composition.

A cyclic lipopeptide biosurfactant is a natural surfactant having a hydrophilic cyclic peptide part and a non-hydrophilic long chain hydrocarbon group. The cyclic peptide part contains 1 or more anionic groups such as a carboxy group and a phenolic hydroxy group. The present invention can provide the gelatinous composition and the emulsified composition excellent in both of a use feeling and an emulsion stability by the combination of a phospholipid and a cyclic lipopeptide biosurfactant. The gelatinous composition and the emulsified composition of the present invention is stable even in the case where the content amount of an oily ingredient is relatively small, since a cyclic lipopeptide biosurfactant may form a network by the interaction between a side chain carboxy group and a hydroxy group in the peptide part due to a hydrogen bond and the oily ingredient may be dispersed in the network to be stable.

An example of the cyclic lipopeptide biosurfactant includes 1 or more cyclic lipopeptide biosurfactants selected from surfactin, arthrofactin, iturin and salts thereof, and the cyclic lipopeptide biosurfactant is preferably surfactin or a salt thereof.

The surfactin salt is a compound represented by the general formula (II) or a composition containing 2 or more of the compounds. 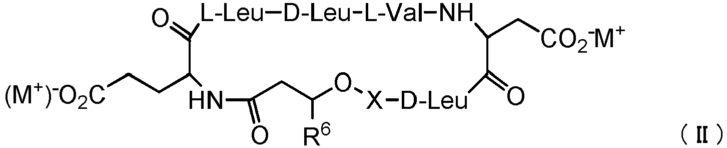 wherein X is an amino acid residue selected from leucine, isoleucine and valine; R⁶ is a C₉₋₁₈ alkyl group; M⁺ is an alkali metal ion or a quaternary ammonium ion.

The amino acid residue as 'X' may be either in an L-form or a D-form, and the L-form is preferred.

The "C₉₋₁₈ alkyl group" means a linear or branched monovalent saturated hydrocarbon group having a carbon number of 9 or more and 18 or less. An example of the C₉₋₁₈ alkyl group includes n-nonyl, 6-methyloctyl, 7-methyloctyl, n-decyl, 8-methylnonyl, n-undecyl, 9-methyldecyl, n-dodecyl, 10-methylundecyl, n-tridecyl, 11-methyldodecyl, n-tetradecyl, n-pentadecyl, n-hexadecyl, n-heptadecyl and n-octadecyl.

The alkali metal ion is not particularly restricted, is exemplified by a lithium ion, a sodium ion and a potassium ion, and is preferably a sodium ion.

An example of a substituent of the quaternary ammonium ion includes an organic group exemplified by an alkyl group such as methyl, ethyl, n-propyl, isopropyl, n-butyl and tert-butyl; an aralkyl group such as benzyl, methylbenzyl and phenylethyl; and an aryl group such as phenyl, toluyl and xylyl. An example of the quaternary ammonium ion includes a tetramethylammonium ion, a tetraethylammonium ion and a pyridinium ion.

Arthrofactin is represented by the general formula (III).

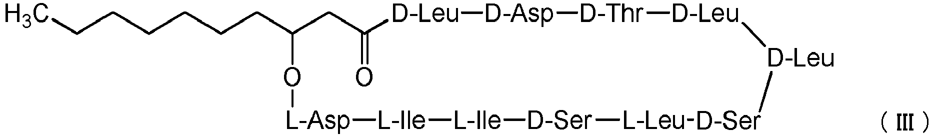

Arthrofactin has one D-aspartic acid and one L-aspartic acid respectively in the structure, which may form a salt with an alkali metal ion and a quaternary ammonium ion.

Iturin is represented by the general formula (IV).

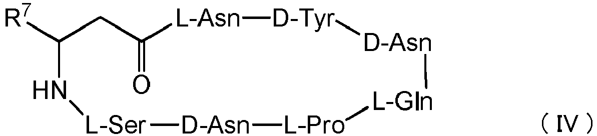

In the formula (IV), R⁷ is a C₉₋₁₈ alkyl group such as -(CH₂)₁₀CH₃, -(CH₂)₈CH(CH₃)CH₂CH₃ and -(CH₂)₉CH(CH₃)₂.

One kind of or two or more kinds of the cyclic lipopeptide biosurfactant or salt thereof may be used. The cyclic lipopeptide biosurfactant can be isolated from a culture medium prepared by cultivating a microorganism that produces the target cyclic lipopeptide biosurfactant in accordance with a known method. The cyclic lipopeptide biosurfactant may be a purified product or an unpurified product. Such an unpurified product is exemplified by the culture medium as it is. An example of a microorganism that produces surfactin includes a strain belonging to Bacillus subtilis. The cyclic lipopeptide biosurfactant produced by a chemical synthesis method may be similarly used.

A total ratio of the phospholipid and the cyclic lipopeptide biosurfactant in the gelatinous composition is preferably adjusted to 0.01 mass% or more and 10 mass% or less.

In addition, a mass ratio of the cyclic lipopeptide biosurfactant to the phospholipid is adjusted to 0.25 or more in the present invention. When the mass ratio is 0.25 or more, the gelatinous composition and the emulsified composition excellent in an emulsion stability can be obtained. On the one hand, the mass ratio is preferably 10 or less. When the mass ratio is 10 or less, the gelatinous composition and the emulsified composition excellent in a use feeling can be obtained. The mass ratio is more preferably 8 or less and even more preferably 5 or less.

A surfactant is dissolved using a polyol or a mixed solvent of water and a polyol as a solvent in this step. The "polyol" means an organic compound having 2 or more hydroxy groups in the molecule. An example of the polyol includes an aliphatic diol such as ethylene glycol, 1,2-propanediol, 1,3-propanediol, 1,4-butanediol, 1,5-pentanediol, 1,6-hexanediol, 1,7-heptanediol, 1,8-octanediol, neopentyl glycol and 1,4-butenediol; and a tri- or more valent polyol such as glycerin, diglycerin, triglycerin, pentaerythritol, trimethylolpropane, sorbitol and xylitol. The polyol is preferably glycerin or a mixture of glycerin and a polyol other than glycerin. An example of such a mixture includes a mixture of glycerin and a polyol selected from diglycerin, sorbitol and xylitol. When glycerin is mixed with other polyol to be used, ratios thereof may be appropriately adjusted. For example, a ratio of the other polyol to the total of glycerin and the other polyol may be adjusted to 1 mass% or more and 95 mass% or less, though the ratio is dependent on whether the polyol other than glycerin is a solid or a liquid at an atmospheric temperature.

In particular, when a polyol that is a solid under an atmospheric temperature and an atmospheric pressure is used, a mixed solvent of water and a polyol is preferably used. When a polyol that is a liquid under an atmospheric temperature and an atmospheric pressure is used, a mixed solvent of water and a polyol may be also used. A concentration of the polyol in the mixed solvent is not particularly restricted and may be appropriately adjusted to, for example, 5 mass% or more and 95 mass% or less.

A condition to prepare a surfactant solution may be appropriately adjusted. For example, the phospholipid and the cyclic lipopeptide biosurfactant are added to a polyol or the above-described mixed solvent and the mixture may be merely stirred. When at least one of the phospholipid and the cyclic lipopeptide biosurfactant is hardly dissolved, the polyol may be appropriately selected, ratios of water and the polyol may be adjusted, and the mixture may be warmed at about 40°C or higher and 90°C or lower.

### 2. Step for producing gelatinous composition

The surfactant solution prepared in the above-described Step 1 and an oily ingredient are mixed to produce the gelatinous composition in this step. The gelatinous composition has a viscosity of 3000 mPa·s or more, and the viscosity is measured in accordance with JIS Z 8803: 2011.

The oily component usable in the present invention is not particularly restricted as long as the oily component cannot be miscible with water in any proportions. The oily component specifically means a substance that is not dissolved within 30 minutes when 1 g or 1 mL of the substance is added to 1000 mL or more of water and the mixture is strongly shaken at 20±5°C for 30 seconds every 5 minutes. An example of the oily component includes a hydrocarbon such as squalane, liquid paraffin, light liquid isoparaffin, ceresin, polyethylene powder, squalene, microcrystalline wax, petrolatum, liquid isoparaffin, polybutene and mineral oil; a wax such as beeswax, carnauba wax, candelilla wax, jojoba oil, lanolin and spermaceti; a fat and oil such as macadamia nut oil, olive oil, cottonseed oil, soybean oil, avocado oil, rice bran oil, rice oil, rice germ oil, palm kernel oil, castor oil, rose hip oil, evening primrose oil, camellia oil, horse oil, grape seed oil, palm oil, meadow foam oil, shea butter, corn oil, safflower oil and sesame oil; an ester such as ethylhexyl palmitate, isononyl isononanoate, isopropyl myristate, ethyl oleate, glycerol tri-(caprylate/caprate), cetyl 2-ethylhexanoate, glyceryl tri(2-ethylhexanoate), diisopropyl sebacate and cholesteryl hydroxystearate; a long chain fatty acid such as myristic acid, stearic acid and oleic acid; a silicone oil such as polymethylsiloxane, polymethylphenylsiloxane and an amino-modified silicone; a higher alcohol such as cetanol and oleyl alcohol; an alkyl glyceryl ether such as batyl alcohol and chimyl alcohol.

A ratio of the oily ingredient in the gelatinous composition may be appropriately adjusted and may be adjusted to, for example, 1 mass% or more and 90 mass% or less. The ratio is preferably 80 mass% or less and more preferably 75 mass% or less. In the case of a prior art gelatinous composition, especially when an amount of an oily ingredient is relatively small, the oil phase and the aqueous phase may separate and the gel state may not be maintained stably in some cases. On the one hand, the gel state of the gelatinous composition according to the present invention can be stably maintained particularly by the function of the cyclic lipopeptide biosurfactant even when an amount of the oily ingredient is relatively small. The ratio is preferably less than 50 mass%, more preferably 40 mass% or less and even more preferably less than 40 mass% from the above standpoint.

A condition to produce the gelatinous composition is not particularly restricted. For example, the oily ingredient may be added to the stirred surfactant solution in small batches intermittently or continuously. After a whole amount of the oily ingredient is added, it is preferred to sufficiently stir or mix the mixture so that the gelatinous composition becomes homogenous.

### 3. Step for producing emulsified composition

Then, a method for producing the emulsified composition of the present invention is described with the specific embodiment, but the present invention is not restricted to the following embodiment. The gelatinous composition produced in the above-described Step 2 is dispersed in an aqueous solvent to produce the emulsified composition in this step.

An example of an aqueous solvent in which the gelatinous composition is dispersed in the present invention includes a mixed solvent of water and a water-miscible organic solvent in addition to water. A water-miscible organic solvent means an organic solvent that can be unrestrictedly immixed with water and exemplified by a C₁₋₄ alcohol such as ethanol and isopropanol. A ratio of a water-miscible organic solvent in the mixed solvent of water and a water-miscible organic solvent may be appropriately adjusted, may be adjusted to, for example, 0.1 mass% or more and 20 mass% or less, and is preferably 0.5 mass% or more and 1 mass% or less, and preferably 15 mass% or less or 10 mass% or less, more preferably 5 mass% or less.

An amount of the usable aqueous solution may be appropriately adjusted and for example, a total ratio of the phospholipid and the cyclic lipopeptide biosurfactant to the emulsified composition may be adjusted to 0.01 mass% or more and 10 mass% or less.

When an emulsified composition is produced by a mechanical stirring method, a strong stirring power and a strong shearing force are needed. On the one hand, the emulsified composition can be produced even by a relatively weak stirring power and shearing force. For example, the emulsified composition can be produced even with a weak stirring power of 500 rpm or less by using a disperser mixer or a puddle mixer without any trouble. The stirring condition is not particularly restricted as long as the mixture can be homogenously dispersed.

When other component is mixed with the emulsified composition of the present invention, the other component may be added in any one or more steps of the above-described Step 1 for producing the surfactant solution, the above-described Step 2 for producing the gelatinous composition and this step for producing the emulsified composition. An optional ingredient that may be added to the emulsified composition of the present invention other than the above-described phospholipid, cyclic lipopeptide biosurfactant, polyol, oily ingredient and aqueous solvent as essential components is exemplified by a C₁₋₄ alcohol such as ethanol and isopropanol; and a thickener, a ultraviolet absorber, an antioxidant, an emollient agent, a solubilizer, an anti-inflammatory drug, a moisturizer, a preservative, a disinfectant, a dye, a fragrance and a powder.

The gelatinous composition and the emulsified composition of the present invention are excellent in both of an emulsion stability and a use feeling, since the gelatinous composition and the emulsified composition contain the phospholipid and the cyclic lipopeptide biosurfactant in the specific ratios. The gelatinous composition and the emulsified composition of the present invention are, therefore, particularly useful as cosmetics and a skin external agent, which are directly applied on the skin.

The present application claims the benefit of the priority date of Japanese patent application No. 2019-18630 filed on February 5, 2019. All of the contents of the Japanese patent application No. 2019-18630 filed on February 5, 2019, are incorporated by reference herein.

### EXAMPLES

Hereinafter, the examples are described to demonstrate the present invention more specifically, but the present invention is in no way restricted by the examples, and the examples can be appropriately modified to be carried out within a range that adapts to the contents of this specification. Such a modified example is also included in the scope of the present invention.

### Examples 1 to 5: Production of emulsified composition

Sodium surfactin and hydrogenated lecithin manufactured by Tsuji Oil Mills were added to glycerin in mass ratios shown in Table 1, and the mixture was stirred at an atmospheric temperature to be dissolved. Squalane was gradually added to the obtained solution while the solution was slowly stirred by hand using a stirring bar at an atmospheric temperature to obtain a gelatinous composition.

Then, the gelatinous composition was added to purified water in mass ratios shown in Table 1, and the mixture was slowly stirred by hand using a stirring bar at an atmospheric temperature to be completely homogeneously dispersed to obtain an emulsified composition.

Comparative example 1: Production of emulsified composition Only hydrogenated lecithin manufactured by Tsuji Oil Mills was added to glycerin in mass ratios shown in Table 1, and the mixture was stirred at an atmospheric temperature to be dissolved. The obtained solution was slowly stirred by hand using a stirring bar at an atmospheric temperature to be completely homogeneously dispersed, and squalane was gradually added thereto to obtain a gelatinous composition.

Then, the gelatinous composition was added to purified water in mass ratios shown in Table 1, and the mixture was slowly stirred by hand using a stirring bar at an atmospheric temperature to be completely homogeneously dispersed to obtain an emulsified composition.

**Table 1**

| | Ex. 1 | Ex. 2 | Ex. 3 | Ex. 4 | Ex. 5 | Comparative ex. 1 |
|---|---|---|---|---|---|---|
| Sodium surfactin | 0.8 | 0.7 | 0.5 | 0.3 | 0.2 | 0 |
| Hydrogenerated lecithin | 0.2 | 0.3 | 0.5 | 0.7 | 0.8 | 1 |
| Glycerin | 15 | 15 | 15 | 15 | 15 | 15 |
| Squalane | 8 | 8 | 8 | 8 | 8 | 8 |
| Purified water | 76 | 76 | 76 | 76 | 76 | 76 |
| Total | 100 | 100 | 100 | 100 | 100 | 100 |
| Sodium surfactin/ Hydrogenerated lecithin | 4 | 2.3 | 1 | 0.42 | 0.25 | 0 |

### Test example 1: Use feeling evaluation

About 1 g of the emulsified compositions of Examples 1 to 5 and Comparative example 1 were applied on the inside of upper arm, the inside of the wrist or the back of the hand of 6 test subjects, and a use feeling was evaluated on the following 5-grade. The result is shown in Table 2. The "Tenderness" means a feeling that the surface of the skin became softer due to application.

**Table 2**

| | Ex. 1 | Ex. 2 | Ex. 3 | Ex. 4 | Ex. 5 | Comparative ex. 1 |
|---|---|---|---|---|---|---|
| Moist feeling | 4 | 5 | 5 | 5 | 5 | 5 |
| Affinity to skin | 5 | 5 | 5 | 5 | 5 | 5 |
| Tenderness | 4 | 4 | 5 | 5 | 5 | 5 |

As the result shown in Table 2, any emulsified compositions of Examples 1 to 5 and Comparative example 1 are excellent in a use feeling to the skin. The reason may be considered to be that the emulsified compositions contain 0.2 mass% or more of hydrogenated lecithin.

### Test example 2: Emulsion stability evaluation

After the emulsified compositions of Examples 1 to 5 and Comparative example 1 were left to stand at 50°C for 3 days, the appearance was observed to evaluate an emulsion stability on the following criteria. The result is shown in Table 3.
Good: Emulsified state was maintained
Bad: Emulsified state became worse and oil ingredient was separated

**Table 3**

| | Ex. 1 | Ex. 2 | Ex. 3 | Ex. 4 | Ex. 5 | Comparative ex. 1 |
|---|---|---|---|---|---|---|
| Immediately following production | Good | Good | Good | Good | Good | Good |
| 3 days after | Good | Good | Good | Good | Good | Bad |

As the result shown in Table 3, when the emulsified composition of Comparative example 1 that did not contain hydrogenated lecithin as a phospholipid was left to stand at 50°C for 3 days, the emulsified composition became separated. On the one hand, the emulsified state of the emulsified compositions of Examples 1 to 5 that contained both of hydrogenated lecithin and surfactin was maintained.

The reason why the emulsified composition of the present invention is excellent in stability may be that the emulsified composition contains surfactin in addition to a phospholipid as surfactants and the surfactin forms a network structure due to a hydrogen bond interaction between the side chain carboxy groups; as a result, even if a ratio of an oily ingredient is relatively small, the oily ingredient is dispersed in the network structure to be stable.

### Test example 3: Emulsification capacity evaluation

Sodium surfactin and hydrogenated lecithin manufactured by Tsuji Oil Mills or only hydrogenated lecithin was added to glycerin in mass ratios shown in Table 4, and the mixture was stirred at an atmospheric temperature to be dissolved.

**Table 4**

| | Ex. 6 | Ex. 7 | Ex. 8 | Ex. 9 | Ex. 10 | Comparative ex. 2 |
|---|---|---|---|---|---|---|
| Sodium surfactin | 0.8 | 0.7 | 0.5 | 0.3 | 0.2 | 0 |
| Hydrogenerated lecithin | 0.2 | 0.3 | 0.5 | 0.7 | 0.8 | 1 |
| Glycerin | 20 | 20 | 20 | 20 | 20 | 20 |
| Sodium surfactin/ Hydrogenerated lecithin | 4 | 2.3 | 1 | 0.42 | 0.25 | 0 |

Then, squalene was gradually added to each stirred solution to produce a gelatinous composition. The stage at which the gel could not be maintained and squalene was separated with adding squalene was judged to be the limit of the emulsification capacity. The result is shown in Table 5. In Table 5, "Good" represents that the gel was maintained, and "Bad" represents that squalene was separated.

**Table 5**

| Additive amount of squalane | Ex. 6 | Ex. 7 | Ex. 8 | Ex. 9 | Ex. 10 | Comparative ex. 2 |
|---|---|---|---|---|---|---|
| 5 wt% | Good | Good | Good | Good | Good | Good |
| 10 wt% | Good | Good | Good | Good | Good | Good |
| 15 wt% | Good | Good | Good | Good | Good | Bad |
| 20 wt% | Good | Good | Good | Good | Good | Bad |
| 50 wt% | Good | Good | Good | Good | Good | Bad |
| 70 wt% | Good | Good | Good | Good | Good | Bad |

As the result shown in Table 5, a sufficient amount of squalene as an oily ingredient could not be dispersed in the composition of Comparative example 2 that did not contain sodium surfactin. The reason may be that the emulsification capacity of hydrogenated lecithin is low.

On the one hand, a sufficient amount of squalene could be dispersed in the compositions of Examples 6 to 10 that contained sodium surfactin in addition to hydrogenated lecithin at a ratio of 0.25 or more of sodium surfactin/hydrogenated lecithin, and the gelatinous composition containing a sufficient amount of squalene could be produced.

It was experimentally demonstrated from the above results that the emulsified composition of the present invention is very excellent in a use feeling and additionally has an excellent capacity to emulsify an oily ingredient by containing the specific biosurfactant and a phospholipid at a ratio of 0.25 or more of biosurfactant/phospholipid.

## Claims

1. A method for producing a gelatinous composition, the method comprising the steps of:
dissolving a phospholipid and a cyclic lipopeptide biosurfactant in a polyol or a mixed solvent of water and a polyol to obtain a surfactant solution, and
mixing an oily ingredient with the surfactant solution to obtain the gelatinous composition,
wherein a mass ratio of the cyclic lipopeptide biosurfactant to the phospholipid in the gelatinous composition is 0.25 or more.

2. The method according to claim 1, wherein the phospholipid is one or more phospholipids selected from phosphatidylcholine, hydrogenated lecithin, phosphatidic acid, bisphosphatidic acid, phosphatidylethanolamine, phosphatidylmethylethanolamine, phosphatidylserine, phosphatidylinositol, phosphatidylglycerol, diphosphatidylglycerol and sphingomyelin.

3. The method according to claim 1 or 2, wherein the cyclic lipopeptide biosurfactant is one or more cyclic lipopeptide biosurfactants selected from surfactin, arthrofactin, iturin and salts thereof.

4. The method according to any one of claims 1 to 3, wherein a total ratio of the phospholipid and the cyclic lipopeptide biosurfactant in the gelatinous composition is 0.01 mass% or more and 10 mass% or less.

5. The method according to any one of claims 1 to 4, wherein a ratio of the oily ingredient in the gelatinous composition is 1 mass% or more and 90 mass% or less.

6. The method according to any one of claims 1 to 4, wherein a ratio of the oily ingredient in the gelatinous composition is 1 mass% or more and less than 50 mass%.

7. A method for producing an emulsified composition, the method comprising the steps of:
producing the gelatinous composition by the method according to any one of claims 1 to 6, and
dispersing the gelatinous composition in an aqueous solvent.

8. A gelatinous composition,
comprising a phospholipid and a cyclic lipopeptide biosurfactant,
wherein a mass ratio of the cyclic lipopeptide biosurfactant to the phospholipid is 0.25 or more.

9. An emulsified composition,
comprising a phospholipid, a cyclic lipopeptide biosurfactant and an aqueous solvent,
wherein a mass ratio of the cyclic lipopeptide biosurfactant to the phospholipid is 0.25 or more.
